# EUROPÄISCHE PATENTANMELDUNG

(11) **EP 3 693 037 A1**
(43) Veröffentlichungstag der Anmeldung: **12.08.2020**
(21) Anmeldenummer: 19155959.0
(22) Anmeldetag: 07.02.2019
(51) Int. Cl.: A61M 1/06, A61M 1/00

(54) **SAUGPUMPE**

(71) Anmelder: Medela Holding AG, 6340 Baar (CH)
(72) Erfinder: Furrer, Etienne, 6330 Cham (CH); Bächler, Cornel, 6038 Honau (CH); Felber, Armin, 6003 Luzern (CH); Hofstetter, Stefan, 6340 Baar (CH); Marbet, Regina, 4933 Rütschelen (CH); Walter, Andreas, 8910 Affoltern am Albis (CH)
(74) Vertreter: Grünecker Patent- und Rechtsanwälte PartG mbB

(57) **Zusammenfassung**

Die vorliegende Erfindung betrifft eine motorgetriebene medizinische Saugpumpe, insbesondere Brustpumpe, mit einem Pumpaggregat (6) zur Erzeugung eines Unterdrucks, das in einem Pumpengehäuse (2, 4) aufgenommen ist. Zur Schaffung einer kostengünstigen, gleichwohl leistungsfähigen aber auch hinsichtlich Geräuschabsonderung und Vibration verbesserten und kompakt bauenden medizinischen Saugpumpe wird mit der vorliegenden Erfindung vorgeschlagen, zumindest zwei Motoren (50) zur Erzeugung des Unterdrucks als Bestandteil des Pumpaggregats (6) vorzusehen. Nach einem alternativen Aspekt der vorliegenden Erfindung ist in dem Pumpengehäuse (2, 4) eine passive Druckausgleichskammer (45) vorgesehen, die mit einer Saugseite des Pumpaggregats (6) kommuniziert.

## Beschreibung

Die vorliegende Erfindung betrifft eine motorgetriebene medizinische Saugpumpe, insbesondere Brustpumpe, mit einem Pumpaggregat zur Erzeugung eines Unterdrucks, das in einem Pumpgehäuse aufgenommen ist.

Eine solche Saugpumpe ist beispielsweise aus US 8,876,760 B2 bekannt.

Die erfindungsgemäße wie auch die vorbekannte Saugpumpe dient insbesondere dem Absaugen von Muttermilch aus der weiblichen Brust. Die erfindungsgemäße Saugpumpe lässt sich aber auch für andere medizinische Zwecke einsetzen, beispielsweise für das Absaugen von Wundsekret aus dem Wundbereich.

Bei dem zuvor erwähnten Stand der Technik befindet sich zwischen dem den Unterdruck erzeugenden Motor und einer Membran, die das Arbeitsmedium des Motors von dem anzusaugenden Fluid trennt, eine Pufferkammer mit variablem Volumen. Die Pufferkammer hat hierzu einen von einem Motor gestellten Kolben, durch den das Volumen des Puffers verändert werden kann, um den an der Membran anliegenden Unterdruck des Arbeitsmediums zu variieren.

Der vorliegenden Erfindung liegt das Problem zugrunde, eine motorgetriebene medizinische Saugpumpe anzugeben, die sich kostengünstig herstellen lässt, möglichst vibrations- bzw. geräuscharm betrieben werden kann und eine kompakte Bauweise ermöglicht.

Zur Lösung dieses Problems schlägt die vorliegende Erfindung eine motorbetriebene medizinische Saugpumpe der eingangs genannten Art vor, bei der das Pumpaggregat zur Erzeugung des Unterdrucks zumindest zwei Motoren aufweist. Die Motoren des erfindungsgemäßen Pumpaggregats sind jeweils Motoren zur Erzeugung des Unterdrucks. Die Motoren haben dafür jeweils ihnen unmittelbar zugeordnete Pumpkammern, deren Volumina durch den Betrieb der Motoren verändert werden können, um eine Saugleistung zu erzielen. Die Pumpkammer wird üblicherweise durch eine bewegliche Membran gebildet, die über einen unmittelbar mit der Motorachse verbundenen Exzenterantrieb zyklisch hin- und herbewegt wird. Jeder Pumpkammer ist ein motorisch angetriebenes Ventil unmittelbar zugeordnet, um Umgebungsluft in die variable Pumpkammer einzuleiten. Die beiden Motoren sind üblicherweise identischer Bauart. Mit anderen Worten sind die einzelnen Komponenten der Motoren einschließlich der Pumpkammern identisch ausgebildet, was eine wirtschaftliche Herstellung der erfindungsgemäßen Saugpumpe begünstigt. Die Pumpkammer, das Einlassventil und der Motor sind üblicherweise zu einer Baugruppe miteinander verbunden. Die Saugöffnung jeder einzelnen, den Motoren zugeordneten Pumpkammer ist üblicherweise mit einem einheitlichen Adapterelement verbunden, welches Schlauchanschlüsse für Schläuche aufweist, die zu einer außen an dem Pumpgehäuse vorgesehenen Saugöffnung führen.

Während die erfindungsgemäße Lösung zumindest zwei Motoren zur Erzeugung des Unterdrucks aufweist, hat die vorbekannte Lösung nach US 8,876,760 B2 lediglich einen solchen Motor mit einer zugeordneten Pumpkammer. Das Arbeitsmedium wird von dieser Pumpkammer in den Puffer eingeleitet, dessen Volumen zwar veränderlich ist. Hier wird lediglich das Niveau des zunächst erzeugten Unterdrucks verändert, ohne dass dem Motor selbst eine den Unterdruck des Arbeitsmediums erzeugende Funktion zukommt.

Die Anordnung von zwei kleineren Motoren statt eines großen Motors bietet Vorteile bei der Ausgestaltung der Saugpumpe. Sie kann vibrationsärmer betrieben werden, da die bewegten Massen von zwei Motoren nicht notwendigerweise synchron bewegt werden. Auch lässt sich die erfindungsgemäße Saugpumpe kostengünstiger herstellen, wenn die beiden Motoren und die ihnen zugeordneten Pumpkammern unter Verwendung gleicher Komponenten hergestellt werden.

Gemäß einem nebengeordneten Aspekt der vorliegenden Erfindung kommuniziert die Saugseite des Pumpaggregats mit einer passiven Druckausgleichskammer, die in dem Pumpengehäuse aufgenommen ist. Eine solche passive Druckausgleichskammer erhöht das in der Saugpumpe vorgesehene Volumen des Arbeitsmediums, sodass diese besser auf variierende Einflüsse reagieren kann, die beispielsweise durch das anzusaugende Fluid eingebracht werden. Systembedingte Variationen des auf der Seite des anzusaugenden Fluids ausgebildeten Volumens oder aber auch spontane Änderungen der dort wirkenden Druckverhältnisse können aufgrund der passiven Druckausgleichskammer durch die erfindungsgemäße Saugpumpe besser kompensiert werden. Die Druckausgleichskammer befindet sich zwischen dem Pumpaggregat und einer Membran, die das Arbeitsmedium von dem anzusaugenden Medium trennt. Das Pumpengehäuse weist bevorzugt eine Saugöffnung auf, die ohne Zwischenschaltung einer Membran die Saugleistung der Saugpumpe an der Außenseite des Pumpengehäuses verfügbar macht.

Die erfindungsgemäße passive Druckausgleichskammer hat ein fest vorgegebenes Volumen. Im Gegensatz zu dem zuvor genannten Stand der Technik nach US 8,876,760 B2 fehlt es an einem aktiven Antrieb zur Veränderung der Lage einer Membran. Das Volumen der Druckausgleichskammer ist fest vorgegeben. Die Wände der Druckausgleichskammer sind nicht motorisch angetrieben. Vorbehaltlich etwaiger Verformungen von die Druckausgleichskammer definierenden Wänden aufgrund sich ändernder Druckverhältnisse sind die Kammerwände als starr anzusehen. Das Volumen der Druckausgleichskammer kann nicht aktiv verändert werden.

Mit Blick auf eine geringere Vibration der Saugpumpe beim Betrieb wird gemäß einer bevorzugten Weiterbildung der vorliegenden Erfindung vorgeschlagen, die Motoren so relativ zueinander anzuordnen, dass sich beim Betrieb der Motoren die freien Massekräfte der Motoren bzw. des Pumpaggregats zumindest teilweise ausgleichen. Ein solcher Ausgleich kann beispielsweise dadurch erzielt werden, dass die beiden Motoren gegenläufig drehend angetrieben werden. So wird in jedem Fall verhindert, dass sich die freien Massenkräfte der einzelnen Motoren verstärken. Dieser positive Effekt zeigt sich im Besonderen beim Anfahren der Saugpumpe. Eine Gestaltung, bei welcher sich die Massekräfte ganz oder teilweise ausgleichen, kann auch dadurch erreicht werden, dass die Pumpkammern auf in Bezug auf die Längsachse der Motoren gegenüberüberliegenden Seiten der einzelnen Motoren angeordnet werden. Die Kolben der Pumpkammern werden bevorzugt im Wesentlichen achsparallel zueinander angeordnet, sind jedoch in Bezug auf die Längsachse der Motoren an gegenüberüberliegenden Seiten angeordnet und/oder so gegenläufig zueinander betrieben, dass sich die Vibrationen bzw. freien Massekräfte der Kolben ausgleichen. Die Motoren können dabei so gesteuert sein, dass diese phasenversetzt betätigt werden. Auch hierdurch ergibt sich eine zumindest teilweise Kompensation von freien Massekräften. In diesem Zusammenhang sind Schrittmotoren zu bevorzugen, die sich insofern exakt ansteuern lassen. Aber auch bei einfacheren Elektromotoren ergibt sich eine verminderte, durch die Antriebe ausgelöste Schwingung, wenn diese zumindest gegenläufig betrieben werden.

Auch im Hinblick darauf wird gemäß einer bevorzugten Weiterbildung der vorliegenden Erfindung vorgeschlagen, die Motorenwellen zumindest angenähert parallel zueinander verlaufen zu lassen. Bei den Motorenwellen handelt es sich dabei üblicherweise um die Antriebsdrehachsen der Antriebsmotoren. Diese Ausgestaltung führt insbesondere bei gegenläufig drehenden Motoren zu einer wirksamen Vibrationsunterdrückung.

Mit Blick auf eine möglichst gute Dämpfung von Schall und Vibrationen sind gemäß einer bevorzugten Weiterbildung der vorliegenden Erfindung die Motoren in einem Pumpaggregatgehäuse aufgenommen. Das Pumpaggregatgehäuse kapselt die Motoren und üblicherweise auch die ihnen jeweils zugeordneten Pumpkammern schalldämpfend ein. Hierzu werden die Motoren durch das Pumpaggregatgehäuse üblicherweise vollumfänglich umgeben. Von den Motoren abgesetzter Lärm kann nicht unmittelbar nach außen dringen. Er wird vielmehr innerhalb des Pumpaggregatgehäuses gefangen.

Die mit dem Betrieb der Motoren einhergehende Vibration und auch das Abdämpfen von Körperschall lässt sich gemäß einer bevorzugten Weiterbildung der vorliegenden Erfindung dadurch besonders abdämpfen, dass das die Motoren aufnehmende Pumpaggregatgehäuse elastisch in dem Pumpengehäuse aufgehängt ist. Die Aufhängung des Pumpaggregatgehäuses erfolgt dabei üblicherweise über bevorzugt lediglich zwei Lagerpunkte. Die Aufhängung ist üblicherweise so ausgestaltet, dass das Pumpaggregatgehäuse translatorische und rotatorische Ausgleichsbewegungen in dem Pumpgehäuse durchführen kann, ohne an die Wandungen des Pumpengehäuses zu stoßen. Der Kontakt erfolgt alleine über Lagerpunkte, die üblicherweise dämpfend bzw. elastisch ausgebildet sind.

Zur Verwirklichung der Aufhängung hat das Pumpaggregatgehäuse bevorzugt von der Außenfläche abragende Lagerzapfen, die jeweils in zugeordneten Abstützungselementen aufgenommen sind. Die Abstützungselemente sind üblicherweise aus einem elastischen Material, wie beispielsweise TPE oder Silikon gebildet. Die Abstützungselemente sind gegenüber dem Pumpengehäuse festgelegt. Die Abstützungselemente haben aber üblicherweise keinen unmittelbaren Kontakt mit den Außenwänden des Pumpgehäuses. Die Lagerzapfen befinden sich üblicherweise auf Höhe des Masseschwerpunktes des Pumpaggregatgehäuses zusammen mit den darin aufgenommenen Komponenten. Es sind üblicherweise an gegenüberliegenden Außenflächen des Pumpaggregatgehäuses Lagerzapfen vorgesehen.

Die die Lagerzapfen verbindende Gerade erstreckt sich dabei bevorzugt schräg zu einer Gerade, die die Motorwellen schneidet. Diese konkrete Gestaltung folgt der allgemeinen Vorgabe, wonach die Motorachsen der Motoren mindestens angenähert parallel zueinander verlaufen und eine die Motorwelle schneidende erste Gerade in einem Winkel zu einer zweiten Geraden verläuft, die zwei Abstützungspunkte verbindet, an denen jeweils ein Abstützungselement außen an dem Pumpaggregatgehäuse angreift. Der von beiden Geraden eingeschlossene Winkel liegt bevorzugt bei zwischen 15° und 90°, besonders bevorzugt bei zwischen 30° und 60°.

Die Abstützungselemente sind üblicherweise in einer Schiebeführung gehalten. Diese Schiebeführung ist teilweise durch ein Gehäuseunterteil und teilweise durch ein Gehäuseoberteil gebildet, die jeweils üblicherweise hauben- oder schalenförmig und als spritzgegossene Kunststoffelemente ausgebildet sind. Die Schiebeführung wird dementsprechend durch eine untere Schiebeführung und eine obere Schiebeführung ausgebildet, die jeweils dem unteren bzw. dem oberen Gehäuseteil zugeordnet sind. Nach Fügen von Gehäuseoberteil und Gehäuseunterteil sind dementsprechend die Abstützungselemente spielfrei in dem Gehäuse über die Schiebeführung aufgenommen. Dabei kann das Abstützungselement beispielsweise in der oberen Schiebeführung vormontiert werden, wobei die untere, an dem Gehäuseunterteil vorgesehene Schiebeführung beim Fügen von Gehäuseoberteil und Gehäuseunterteil über das Abstützungselement gleitet und dieses in Schieberichtung im Grund spielfrei nach der Montage des Gehäuses fixiert.

In Höhenrichtung befindet sich die passive Druckausgleichskammer üblicherweise über dem Pumpaggregat. Dementsprechend ist die Druckausgleichskammer einem oberen Ende des Gehäuseoberteils zugeordnet. Im oberen Teil der auf dem durch das Gehäuseunterteil gebildeten Boden stehenden Saugpumpe befindet sich üblicherweise die Saugöffnung der Saugpumpe. Die Druckausgleichskammer ist dieser Saugöffnung unmittelbar zugeordnet.

Dabei bildet das Gehäuseoberteil die Druckausgleichskammer zumindest teilweise aus. Mit anderen Worten ist zumindest ein Teil der Druckausgleichskammer durch das spritzgegossene Gehäuseoberteil ausgebildet. So kann das Gehäuseoberteil eine Druckausgleichskammerschale ausformen, die durch einen im Wesentlichen planaren und mit einem Anschlussstutzen versehenen Druckausgleichskammerdeckel abgeschlossen ist. Dieser Druckausgleichskammerdeckel ist üblicherweise mittels Ultraschallschweißen oder Laserschweißen gasdicht mit der Druckausgleichskammerschale verbunden, wodurch die passive Druckausgleichskammer fertiggestellt ist.

Die Druckausgleichskammer wird üblicherweise durch mehrere, bevorzugt zwei Komponenten gebildet, die eine feste räumliche Zuordnung zueinander haben. Der zuvor erwähnte Anschlussstutzen ist bevorzugt in einer Gehäuseabdeckung aufgenommen und ragt in diese hinein. Die Gehäuseabdeckung ist bevorzugt aus einem elastischen Material ausgebildet, sodass ein den Anschlussstutzen umgebender und zu der Saugöffnung führender Austrittskanal gegenüber dem Anschlussstutzen abgedichtet ist. Der Austrittskanal wird bevorzugt allein durch die Gehäuseabdeckung ausgeformt, die unmittelbar die Druckausgleichskammer abdeckt.

Das elastische Material der Gehäuseabdeckung erlaubt die Ausformung von einem, in der Regel mehreren Tastenelementen zur Steuerung des Pumpaggregats an der Gehäuseabdeckung. Dazu ist zwischen der Druckausgleichskammer und der Gehäuseabdeckung üblicherweise eine bestückte Leiterplatte (im Folgenden: PCB) vorgesehen, die gegenüberliegend zu den Tastenelementen der Gehäuseabdeckung mit Schaltern versehen ist, sodass die Berührung der Tastenelemente zu einem elektrischen Steuersignal führt.

Die Gehäuseabdeckung ist auf ihrer Außenseite üblicherweise auch mit Blick auf hygienische Anforderungen im Wesentlichen flach und eben ausgebildet. Auf der gegenüberliegenden Innenseite hat die Gehäuseabdeckung üblicherweise Stege, die als elastische Stützstege gegen die Leiterplatte wirken und diese in Richtung auf das Gehäuseoberteil fixieren. Auf der den Stützstegen der Gehäuseabdeckung gegenüberliegenden Seite der Leiterplatte befinden sich Lagersegmente eines PCB-Stützrahmens, der die Leiterplatte hält und fixiert. Dieser PCB-Stützrahmen hat regelmäßig einen aufstehenden ggf. auch unterbrochen umlaufenden Rand, der von einem Halterand der Gehäuseabdeckung umgriffen ist, sodass die elektronischen Komponenten durch die Gehäuseabdeckung nicht nur von oben sondern auch seitlich umschlossen sind. Die Leiterplatte hat üblicherweise Steckkontakte zum elektrischen Anschluss von Kabeln, die zu den einzelnen Motoren führen.

Die mechanische Fixierung der Leiterplatte und der Gehäuseabdeckung erfolgt üblicherweise durch Verrasten des PCB-Stützrahmens gegenüber dem Gehäuseoberteil. Der PCB-Stützrahmen liegt üblicherweise unmittelbar oberhalb der Druckausgleichskammer, bevorzugt liegt er oberseitig gegen die Druckausgleichskammer an. Die Verrastung zwischen der Gehäuseabdeckung und dem PCB-Stützrahmen wird im montierten Zustand üblicherweise durch einen überstehenden Rand des Gehäuseoberteils gesichert, der mit der Außenumfangsfläche des Halterandes der Gehäuseabdeckung bevorzugt ebenfalls eine Rastverbindung eingeht. Hierdurch ist eine Art Labyrinthdichtung zwischen dem oberen Gehäuseelement, der Gehäuseabdeckung und dem PCB-Stützrahmen gebildet, wodurch sicher verhindert wird, dass Feuchtigkeit und Verschmutzung in die Pumpe und insbesondere zu den elektronischen Komponenten der Saugpumpe gelangen.

Die durch die Gehäuseabdeckung überdeckte Leiterplatte kann zumindest eine LED aufweisen, die üblicherweise mittels SMD-Technologie mit der Leiterplatte verbunden ist. Eine solche LED lässt sich zum Ausbringen von optischen Hinweisen oder Befehlen an die Benutzerin der Pumpe nutzen. Die LED wird üblicherweise von einer auf der PCB aufgebrachten Logikeinheit gesteuert.

Weitere Einzelheiten und Vorteile der vorliegenden Erfindung ergeben sich aus der nachfolgenden Beschreibung eines Ausführungsbeispiels in Verbindung mit der Zeichnung. In dieser zeigen:
- Figur 1: eine perspektivische Explosionsdarstellung mit sämtlichen Komponenten des Ausführungsbeispiels;
- Figur 2: eine Längsschnittansicht des Ausführungsbeispiels;
- Figur 3: eine weitere, gegenüber Figur 2 weiter aussen liegende Längsschnittansicht des Ausführungsbeispiels;
- Figur 4: eine Querschnittsansicht des Ausführungsbeispiels durch das Pumpaggregat;
- Figur 5: eine weitere, gegenüber Figur 4 weiter tiefer liegende Querschnittsansicht des Ausführungsbeispiels durch das Pumpaggregat; und
- Figur 6: eine perspektivische Seitenansicht des Ausführungsbeispiels bei abgenommenem Gehäuseunterteil.

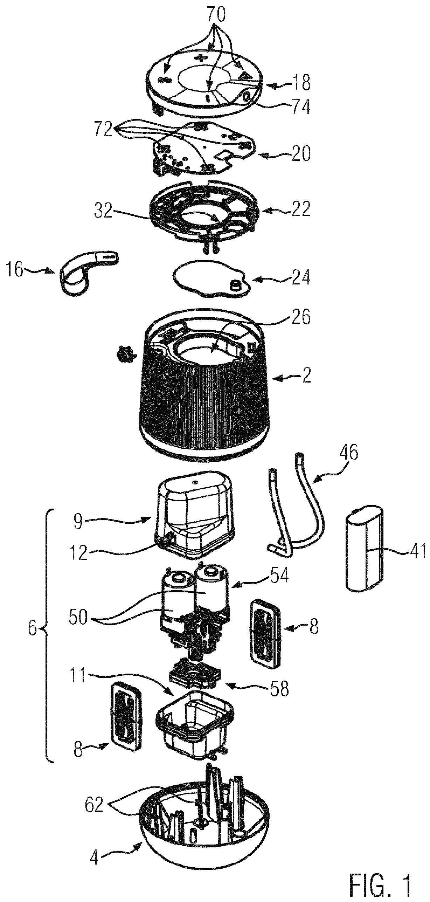

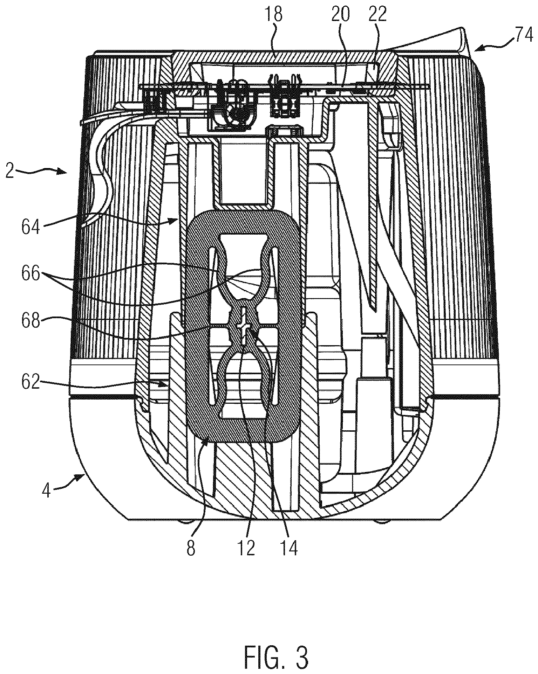

Die Figur 1 zeigt ein Gehäuseoberteil 2 und ein Gehäuseunterteil 4, die zwischen sich ein Pumpaggregat 6 einschließen, welches über Abstützungselemente 8 gegenüber dem Gehäuseoberteil 2 und dem Gehäuseunterteil 4 abgestützt ist. Das Pumpaggregat 6 hat ein aus zwei verrasteten Pumpaggregatgehäuseteilen 9, 11 gebildetes, in den folgenden Figuren mit Bezugszeichen 10 gekennzeichnetes Pumpaggregatgehäuse, das hierzu Lagerzapfen 12 aufweist, die in Zapfenaufnahmen 14 des zugeordneten Abstützungselementes 8 ausgespart sind (vgl. Figur 3). Das Pumpaggregatgehäuse 10 besteht aus Kunststoff.

Von der Außenumfangsfläche des Gehäuseoberteils 2 ragt im montierten Zustand ein textiles Halteband 16 ab, über welches die Saugpumpe getragen werden kann. Mit Bezugszeichen 18 ist eine Gehäuseabdeckung gekennzeichnet, die noch weitere, in Figur 1 gezeigte Komponenten in sich aufnimmt, die mit der Gehäuseabdeckung 18 durch Verrasten verbunden sind. Die Gehäuseabdeckung 18 und die Abstützungselemente 8 bestehen aus Silikon, die übrigen Gehäuseteile 2, 4, 6 aus einem üblichen technischen Kunststoff, beispielsweise PC, PP oder PA.

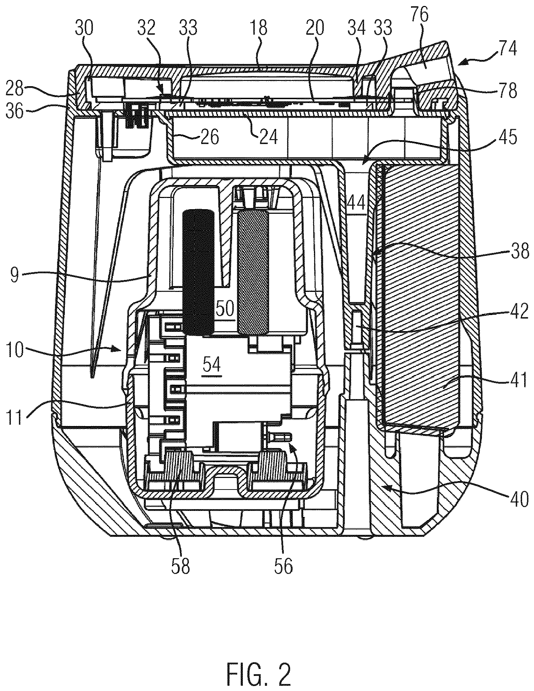

Bei den in der Gehäuseabdeckung 18 aufgenommenen weiteren Komponenten handelt es sich zum einen um eine bestückte Leiterplatte (im Folgenden: PCB) 20 und einen PCB-Stützrahmen 22, welcher auf der der Gehäuseabdeckung 18 gegenüberliegenden Seite der PCB 20 vorgesehen ist. Unterhalb des PCB-Stützrahmens 22 ist in Figur 1 ein Druckausgleichskammerdeckel 24 gezeigt, der mittels Ultraschallschweißen mit dem Gehäuseoberteil 2 verbunden ist. Das Gehäuseoberteil 2 weist eine Druckausgleichskammerschale 26 auf. Diese Druckausgleichskammerschale 26 ist integraler Bestandteil des einteilig spritzgegossenen Gehäuseoberteils 2. Der PCB-Stützrahmen 22 hat in Richtung auf das Gehäuseoberteil 2 vorspringende Rastnasen, die in an dem Gehäuseoberteil 2 ausgebildeten Rastöffnungen verrastet sind. Wie insbesondere Figur 2 verdeutlicht, hat die Gehäuseabdeckung 18 einen umlaufenden Halterand 28, der mit einem Stützrand 30 verriegelt ist, der durch den PCB-Stützrahmen 22 ausgeformt ist. In Figur 2 ersichtlich ist der Umgriff der Gehäuseabdeckung 18 um eine Stufe des PCB-Stützrahmens 22.

Der PCB-Stützrahmen 22 hat eine gitterförmige Ausgestaltung und stützt die PCB 20 an diskreten Stellen ab. Der PCB-Stützrahmen 22 hat einen inneren Stützkranz 32, der auf der Unterseite der PCB 20 vorgesehen ist und als Widerlagersegment 33 zu Stützstegen dient, die einteilig durch die Gehäuseabdeckung 18 ausgeformt und in Figur 2 mit Bezugszeichen 34 gekennzeichnet sind. Durch diese Ausgestaltung wird die PCB 20 mit einer gewissen Vorspannung indes elastisch im oberen Bereich des Gehäuseoberteils 2 gehalten und abgestützt.

Das Gehäuseoberteil 2 hat einen oberen umlaufenden Rand 36, welcher den Halterand 28 der Gehäuseabdeckung 18 umfänglich umgibt und damit eine höhere Dichtigkeit gegenüber Umwelteinflüssen bewirkt, sodass weder Schmutz noch Feuchtigkeit zu der PCB 20 gelangen können. Wie Figur 2 entnommen werden kann, weist die Gehäuseabdeckung 18 an ihrem Halterand 28 eine umlaufende Wulst auf, die an einer korrespondierend hierzu vorgesehenen umlaufenden Nut des oberen umlaufenden Randes 36 im Eingriff ist. Hierdurch wird die Dichtigkeit erhöht und eine mechanische Verriegelung der beiden Teile bewirkt.

Bezugszeichen 41 kennzeichnet eine aufladbare Stromquelle (Akku).

Die Schnittansicht nach Figur 2 lässt erkennen, dass das Gehäuseoberteil 2 einen von dem Boden der Druckausgleichskammerschale 26 abragenden Befestigungsstutzen 38 ausformt, der gegenüberliegend zu einem Befestigungsstutzen 40 des Gehäuseunterteils 4 endet und mit einer Verschraubungsbohrung 42 versehen ist. Auf der der Verschraubungsbohrung 42 gegenüberliegenden Seite umgibt der Befestigungsstutzen 38 ein konisches Kammervolumen 44, welches mit dem Kammervolumen kommuniziert, das zwischen dem Boden der Druckausgleichskammerschale 26 und dem damit verschweißten Druckausgleichskammerdeckel 24 eingeschlossen ist.

Diese beiden Volumina bilden zusammen eine passive Druckausgleichskammer 45 innerhalb des Pumpengehäuses 2, 4 der Saugpumpe, deren Ausgleichsvolumen vorbestimmt und unveränderlich ist.

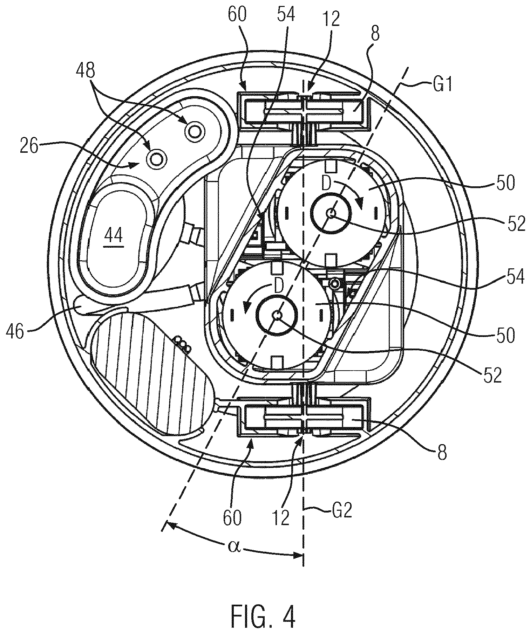

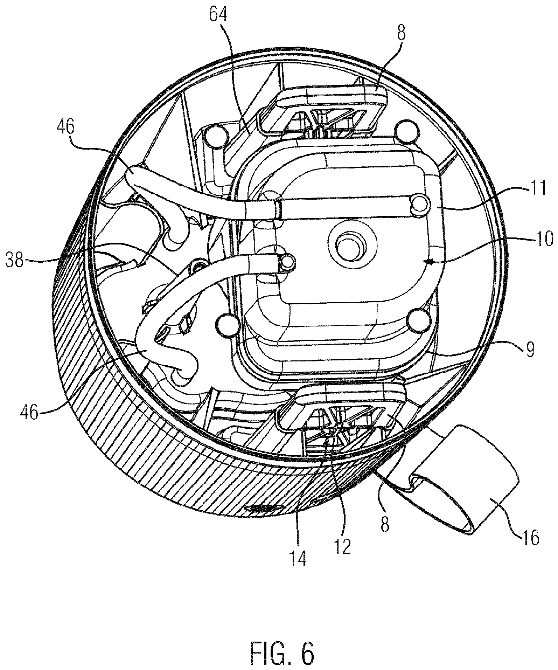

Von der Unterseite des Bodens der Druckausgleichskammerschale 26 ragen Anschlussstutzen ab, die einteilig an dem Gehäuseoberteil 2 angeformt sind und dem Anschluss von zwei Schläuchen 46 dienen. Der genaue Anschluss der Schläuche 46 ergibt sich insbesondere aus Figur 6. Die Schläuche 46 erstrecken sich von der Unterseite des Pumpaggregatgehäuses 10 zu dem Boden der Druckausgleichskammerschale 26. So kommuniziert jeder der Schläuche 46 mit der Druckausgleichskammer 45. Der Zugang der Schläuche 46 zu der Druckausgleichskammer 45 ist in Figur 4 mit Bezugszeichen 48 gekennzeichnet. Über die Schläuche 46 kommuniziert die Saugseite des Pumpaggregats 6 mit der Druckausgleichskammer 45.

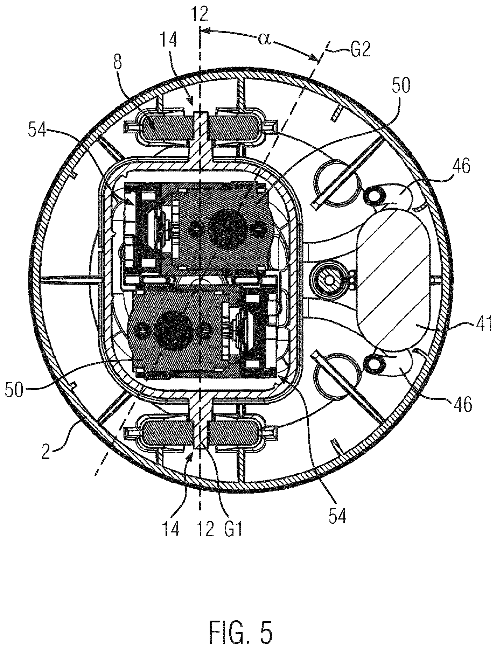

Wie insbesondere die Figuren 4 und 5 vermitteln, hat das Pumpaggregat zwei elektrische Motoren 50, die identisch ausgebildet sind. Mit Bezugszeichen 52 gekennzeichnete Motorenwellen verlaufen parallel zueinander und in Höhenrichtung des Ausführungsbeispiels der Saugpumpe. Die das Gehäuse der Motoren 50 überragenden Wellen 52 sind jeweils mit einer Exzenterscheibe versehen, die umfänglich von einem elastomeren Ring umgeben ist, der einteilig an einer Membran angeformt ist, die als beweglicher Kolben eine Kammer eines Pumpmoduls verschließt. Der äußere Rand des Kolbens ist gehäusefest montiert, so dass lediglich der mittlere Bereich des Kolbens aufgrund der Drehung der Exzenterscheibe zyklisch hin- und herbewegt wird. In der Schnittansicht gemäß Figur 4 und 5 befindet sich das in Figur 2 mit Bezugszeichen 54 gekennzeichnete Pumpmodul im Wesentlichen rechts von dem unteren Motor 50 und links von dem oberen Motor 50. Das Pumpmodul 54 hat ein elektromagnetisches Ventil 56 zum Einlass von Umgebungsluft abhängig von dem Pumpzyklus des Pumpmoduls 54.

Ein in Figur 2 mit Bezugszeichen 58 gekennzeichnetes Adapterelement nimmt die Saugöffnungen der beiden Pumpmodule 54 in sich auf. Das Adapterelement 58 ist aus einem elastischen Material (Silikon) und stützt die Motoren 50 gegenüber dem Pumpaggregatgehäuse 10 dämpfend ab. Das Adapterelement 58 kommuniziert dichtend einerseits jeweils mit der Saugöffnung jedes der Pumpmodule 54 und andererseits mit Schlauchanschlüssen zum Anschluss der Schläuche 46, die außen an dem Pumpaggregatgehäuseteil an dessen Boden angeordnet sind. Die Figuren 4 und 5 verdeutlichen, dass die Pumpmodule 54 und die Motoren 50 jeweils identisch ausgebildet sind. Die mit Bezugszeichen D gekennzeichneten Pfeile verdeutlichen den Drehsinn der Motoren 50 beim Betrieb. Der untere Motor 40 läuft im Gegenuhrzeigersinn; der obere Motor 50 im Uhrzeigersinn. Durch die Anordnung der Pumpmodule 54 an gegenüberliegenden Seiten der Motoren 50 ergibt sich ein Ausgleich der freien Massekräfte des Pumpaggregats. Durch die gegensinnige Drehung der Motoren 50 ergibt sich ein Ausgleich der freien Massenkräfte der Motoren 50. So sind freie Massekräfte des Pumpaggregats beim Betrieb der Motoren 50 zumindest teilweise ausgeglichen.

Die Figuren 3 und 5 verdeutlichen ferner die Aufnahme der Abstützelemente 8 in Schiebeführungen 60. Die Schiebeführungen 60 bestehen für jedes Abstützungselement 8 jeweils aus einer unteren Schiebeführung, die dem Gehäuseunterteil 4 zugeordnet und mit Bezugszeichen 62 gekennzeichnet ist, und einer dem Gehäuseoberteil 2 zugeordneten oberen Schiebeführung 64. Die untere und die obere Schiebeführungen 62, 64 sind jeweils einteilig an dem Gehäuseoberteil bzw. Gehäuseunterteil 2, 4 ausgeformt. Die oberen und unteren Schiebeführungen 62, 64 haben jeweils zwei einander gegenüberliegende U-förmige Aufnahmen, die sich zu ihrem freien Ende hin verjüngen. Der in Höhenrichtung mittlere Teil des Abstützungselementes 8 liegt zwischen den freien Enden der oberen und unteren Schiebeführung 62, 64.

Wie Figur 6 vermittelt, kann das Abstützungselement 8 zunächst bei der Montage in das Gehäuseoberteil 2 eingeschoben werden, sodass das Abstützungselement 8 jeweils in der unteren Schiebeführung 64 aufgenommen ist. Zuvor wurde das Abstützungselement 8 auf die einander gegenüberliegenden Lagerzapfen 12 des Pumpaggregatgehäuses 10 aufgeschoben, sodass das Pumpaggregat 6 über die Zapfenaufnahmen 14 elastisch innerhalb der Gehäuseteile 2, 4 aufgehängt ist. Wie insbesondere Figur 3 vermittelt, erfolgt diese Aufhängung des Pumpaggregatgehäuses 10 so, dass dieses vollumfänglich mit hinreichendem Abstand von Flächen des Gehäuseoberteils 2 bzw. des Gehäuseunterteils 4 vorgesehen ist. So kann das Pumpaggregatgehäuse 10 in Grenzen translatorisch bzw. rotatorisch Ausgleichsbewegungen zum Ausgleich von inneren Schwingungen vollziehen, ohne gegen das Pumpengehäuse 2, 4 zu stoßen.

Figur 3 lässt Details des Abstützungselementes 8 erkennen. Dieses besteht aus einem Rahmen mit orthogonal zueinander sich erstreckenden Schenkeln, in dem diagonale Stege 66 zu der Zapfenaufnahme 14 führen und diese umfänglich umgeben. Von den sich in Höhenrichtung erstreckenden Längsschenkeln des Rahmens erstrecken sich ferner Querstege 68, die ebenfalls an die Zapfenaufnahme 14 anbinden. Durch das elastische Material und diese konkrete Ausgestaltung des Abstützungselementes 8 ergibt sich eine gute Beweglichkeit und Dämpfung des Pumpaggregatgehäuses 10 zum Ausgleich von Schwingungen beim Betrieb der Motoren 50. Das Abstützungselementes 8 ist zwischen dem Gehäuseoberteil 2 und dem Gehäuseunterteil 4 in Höhenrichtung geklemmt und damit fixiert.

Die Gehäuseabdeckung 18 hat verschiedene Funktionen. Wie die Figur 1 erkennen lässt, bildet die elastische Gehäuseabdeckung 18 mehrere Tastenelemente 70 aus. Die Beweglichkeit der Tastenelemente 70 ergibt sich aufgrund der elastischen Materialeigenschaften des Silikons der Gehäuseabdeckung 18 einerseits und dem dünnwandigen Aufbau der Außenfläche der Gehäuseabdeckung 18 andererseits. Unterhalb der Tastenelemente 70 befinden sich jeweils Tastschalter, die in Figur 2 zu erkennen und dort mit Bezugszeichen 72 versehen sind. So kann ein Benutzer der Saugpumpe oben auf die Gehäuseabdeckung 18 an in Umfangsrichtung voneinander beabstandeten und auf der Außenfläche der Gehäuseabdeckung 18 durch Symbole gekennzeichnete Stellen drücken, um bestimmte Steuerbefehle für die Steuerung des Pumpaggregates 6 einzugeben.

Die Gehäuseabdeckung 18 bildet ferner eine in den Figuren 1 und 3 mit Bezugszeichen 74 gekennzeichnete Saugöffnung zum Anschluss an einen Saugschlauch. Dieser Saugschlauch kann eine Luerkanüle aufweisen, die in einen mit Bezugszeichen 76 gekennzeichneten Austrittskanal eingesteckt werden kann. Durch die elastischen Eigenschaften der Gehäuseabdeckung 18 ergibt sich hierbei unmittelbar eine Abdichtung. Diese elastischen Eigenschaften sorgen auch für eine fluiddichte Verbindung zwischen der Gehäuseabdeckung 18 und dem Druckausgleichskammerdeckel 24, der einen in Figur 2 mit Bezugszeichen 78 gekennzeichneten Anschlussstutzen einteilig ausbildet, der durch den Umgriff eines Endes des Austrittskanals 76 dichtend in die Gehäuseabdeckung 18 eingepasst ist.

Die Figur 4 verdeutlicht die Beziehung einer die beiden Motorwellen 52 verbindenden ersten Gerade G1 zu einer zweiten Geraden G2, die zwei Abstützungspunkte verbindet, die vorliegend durch die Lagerzapfen 12 gebildet sind. Eine solche schräge Abstützung hat sich als vorteilhaft zur weiteren Dämpfung des Pumpaggregats 6 gegenüber dem Pumpengehäuse 4, 6 erwiesen. Der von den beiden Gerade G1, G2 eingeschlossene Winkel α liegt vorliegend bei etwa 35°. Er sollte zumindest 15°, aber nicht mehr als 90° betragen. Idealerweise liegt der Winkel zwischen 20° und 70°, bevorzugt zwischen 30° und 60°.

### Bezugszeichenliste

- 2: Gehäuseoberteil
- 4: Gehäuseunterteil
- 6: Pumpaggregat
- 8: Abstützungselement
- 9: Pumpaggregatgehäuseteil
- 10: Pumpaggregatgehäuse
- 11: Pumpaggregatgehäuseteil
- 12: Lagerzapfen
- 14: Zapfenaufnahme
- 16: textiles Halteband
- 18: Gehäuseabdeckung
- 20: PCB
- 22: PCB-Stützrahmen
- 24: Druckausgleichskammerdeckel
- 26: Druckausgleichkammerschale
- 28: Halterand
- 30: Stützrand des PCB-Stützrahmens 22
- 32: Stützkranz
- 33: Widerlagersegment
- 34: Stützsteg
- 36: oberer umlaufender Rand des Gehäuseoberteils 2
- 38: Befestigungsstutzen des Gehäuseoberteils 2
- 40: Befestigungsstutzen des Gehäuseunterteiles 4
- 42: Verschraubungsbohrung
- 41: Akku
- 44: Kammervolumen
- 45: Druckausgleichskammer
- 46: Schlauch
- 48: Zugang
- 50: Motor
- 52: Motorwelle
- 54: Pumpmodul
- 56: Ventil
- 58: Adapterelement
- 60: Schiebeführung
- 62: untere Schiebeführung
- 64: obere Schiebeführung
- 66: Steg
- 68: Quersteg
- 70: Tastenelement
- 72: Tastschalter
- 74: Saugöffnung
- 76: Austrittskanal
- 78: Anschlussstutzen

## Patentansprüche

1. Motorgetriebene medizinische Saugpumpe, insbesondere Brustpumpe, mit einem Pumpaggregat (6) zur Erzeugung eines Unterdrucks, das in einem Pumpengehäuse (2, 4) aufgenommen ist,
**dadurch gekennzeichnet, dass** das Pumpaggregat (6) zur Erzeugung des Unterdrucks zumindest zwei Motoren (50) aufweist.

2. Motorgetriebene medizinische Saugpumpe nach Anspruch 1, **dadurch gekennzeichnet, dass** die Motoren (50) so relativ zueinander angeordnet sind, dass sich beim Betrieb der Motoren (50) die freien Massekräfte des Pumpaggregats (6) zumindest teilweise ausgleichen.

3. Motorgetriebene medizinische Saugpumpe nach Anspruch 1 oder 2, **dadurch gekennzeichnet, dass** die Motorenwellen (52) der Motoren (50) mindestens angenähert parallel zueinander verlaufen.

4. Motorgetriebene medizinische Saugpumpe nach einem der vorherigen Ansprüche, **gekennzeichnet durch** ein die Motoren (50) aufnehmendes Pumpaggregatgehäuse (10), das elastisch in dem Pumpengehäuse (2, 4) aufgehängt ist.

5. Motorgetriebene medizinische Saugpumpe nach einem der vorherigen Ansprüche, **dadurch gekennzeichnet, dass** die Motoren (50) in dem Pumpaggregatgehäuse (10) eingekapselt sind.

6. Motorgetriebene medizinische Saugpumpe nach einem der vorherigen Ansprüche, **dadurch gekennzeichnet, dass** die Motorenwellen (52) der Motoren (50) mindestens angenähert parallel zueinander verlaufen und eine die Motorenwelle (52) schneidende erste Gerade (G1) in einem Winkel (α) zu einer zweiten Gerade (G2) verläuft, die zwei Abstützungspunkte (12) verbindet, an denen jeweils ein Abstützungselement (8) außen an dem Pumpaggregatgehäuse (10) angreift.

7. Motorgetriebene medizinische Saugpumpe nach einem der vorherigen Ansprüche, **dadurch gekennzeichnet, dass** von der Außenfläche des Pumpaggregatgehäuses (10) Lagerzapfen (12) abragen, die jeweils in an dem zugeordneten Abstützungselement (8) ausgesparte Zapfenaufnahmen (14) eingreifen.

8. Motorgetriebene medizinische Saugpumpe nach einem der vorherigen Ansprüche, **dadurch gekennzeichnet, dass** das Abstützungselement (8) zwischen einer Innenfläche des Gehäuses (2, 4) und einer Außenfläche des Pumpaggregatgehäuses (10) mit Abstand zu der Innenfläche des Gehäuses (2, 4) angeordnet ist.

9. Motorgetriebene medizinische Saugpumpe nach einem der vorherigen Ansprüche, **gekennzeichnet durch** eine Schiebeführung (60) zum Halten des Abstützungselementes (8).

10. Motorgetriebene medizinische Saugpumpe nach Anspruch 9, **dadurch gekennzeichnet, dass** die Schiebeführung (60) teilweise an dem Gehäuseunterteil (4) und teilweise an dem Gehäuseoberteil (2) ausgebildet ist.

11. Motorgetriebene medizinische Saugpumpe, insbesondere Brustpumpe, mit einem Pumpaggregat (6) zur Erzeugung eines Unterdrucks, das in einem Pumpengehäuse (2, 4) aufgenommen ist, insbesondere nach einem der Ansprüche 1 bis 10, **gekennzeichnet durch**
eine in dem Pumpengehäuse (2, 4) aufgenommene passive Druckausgleichskammer (45), die mit einer Saugseite des Pumpaggregats (6) kommuniziert.

12. Motorgetriebene medizinische Saugpumpe nach Anspruch 11, **dadurch gekennzeichnet, dass** das Pumpengehäuse (2, 4) ein eine Standfläche des Gehäuses ausbildendes Gehäuseunterteil (4) und ein das Pumpengehäuse oberseitig abschließendes Gehäuseoberteil (2) aufweist, die zwischen sich das Pumpaggregat (6) einschließen, wobei die Druckausgleichskammer (45) einem oberen Ende des Gehäuseoberteils (2) zugeordnet und über dem Pumpaggregat (6) vorgesehen ist.

13. Motorgetriebene medizinische Saugpumpe nach Anspruch 12, **dadurch gekennzeichnet, dass** die Druckausgleichskammer (45) zumindest teilweise durch das Gehäuseoberteil (2) ausgeformt ist.

14. Motorgetriebene medizinische Saugpumpe nach Anspruch 13, **gekennzeichnet durch** eine Gehäuseabdeckung (18) aus einem elastischen Kunststoff, die zumindest ein Tastenelement (70) zur Steuerung des Pumpaggregats (6) ausbildet.
